# EUROPEAN PATENT APPLICATION

(11) **EP 2 294 969 A1**
(43) Date of publication of application: **16.03.2011**
(21) Application number: 09170096.3
(22) Date of filing: 11.09.2009
(51) Int. Cl.: A61B 3/12

(54) **Device for eye fixation**

(71) Applicant: Medical Workshop B.V., 9722 AM Groningen (NL)
(72) Inventor: Groenleer, R.J.J., 1752 AE, Sint Maartensburg (NL)
(74) Representative: 't Jong, Bastiaan Jacob

(57) **Abstract**

The invention relates to a device for eye fixation, the device comprising:
- a screen arrangable in front of an eye of a patient;
- a controller for controlling the screen, wherein the screen is controlled to show a spot at a desired position for a patient's eye to fixate on.

The invention also relates to a headrest comprising:
- a stand;
- a vertical frame arranged on the stand;
- a chin support arranged in the frame for supporting a patient's head; and
- a device for eye fixation according to the invention, mounted to the vertical frame and positionable in front of one of the eye's of a patient.

## Description

The invention relates to a device for eye fixation.

The field of optometry is concerned with performing measurements of the eye, detecting diseases of the eye and taking measurements for contact lenses and glasses. To this end measurement devices are used like for example a fundus camera and a slit lamp.

A fundus camera is a microscope with a camera, which is used to make photos of the inner surface of the eye. This is used for examining the fundus of the eye, which contains for example the retina, the optic disc and the macula.

The slit camera is used to project a thin sheet of light into the eye. In combination with a microscope it is possible to examine the frontal structure of the eye, which comprises for example the sclera, the iris and the cornea.

To be able to perform the examination correctly with such devices, it is necessary that the patient keeps the eyes still. This proves to be difficult while the non examined eye tends to be distracted. As the movement of both eyes is linked, the distraction of the non examined eye results in movement of the eye being examined. The distraction of the non examined eye could be by anything present in the examination room and within eyesight.

In order to prevent undesired movement of the eyes, it is known in the prior art to arrange a light in front of the non examined eye. This light is arranged on a movable arm. During examination the patient is asked to keep the non examined eye directed to the light. When the doctor would like to examine the eye in another position, the doctor has to move the light manually.

When using a fundus camera it is common to let the eye rotate and to make photo's at several positions of the eye. When using a light on a movable arm, which is positioned manually, it is difficult to have the positions at equal distance. If the photo's are made to compare with a previous examination, it is even more difficult to position the light at the same positions as the previous examination. Furthermore, the doctor has to look up from the microscope each time the light has to be repositioned.

It is now an object of the invention to provide a device with which the above mentioned disadvantages is reduced.

This object is achieved with a device according to the invention comprising:
- a screen arrangable in front of an eye of a patient;
- a controller for controlling the screen, wherein the screen is controlled to show a spot at a desired position for a patient's eye to fixate on.

With the screen it is possible to have the spot on a controlled position in front of the eye of the patient. The controller shows a spot on the screen at a desired position, which will attract the non examined eye and makes sure that the eyes are in a desired position. When the doctor would like to move the eyes to another position, the doctor operates the controller such that the spot is moved to another position on the screen, resulting in another position of the eyes.

Furthermore, the screen is arranged in front of the non examined eye and accordingly blocks the view to objects in the examination room. The only thing viewable by the non examined eye is the screen with the spot. This reduces the chance that the patient is distracted. In an embodiment of the device according to the invention the screen comprises a number of light sources connected to the controller for switching on and off the light sources. A light source provides a good contrast with the screen and is good distinguishable by the eye.

A preferred embodiment of the device according to the invention comprises a number of optical fibers, which each have one end arranged in the screen and the other end connected to a light source arranged in the controller. The advantage is that only non electrical elements of the device are present near the eyes of the patient and that all electric components can be arranged at a distance, preferably in the controller. Furthermore, optical fibers are more robust than electrical wires running from the controller to the light sources in the screen.

Another embodiment of the device according to the invention comprises a number of LED's arranged in the screen and connected to the controller. The LED's are a low cost solution for an embodiment of the device according to the invention.

In another preferred embodiment of the device according to invention the screen is concave. On the one hand this accommodates the shape of the eye ball. On the other hand this makes sure that the distance of the spot to the eye is at each position substantially the same. As a result, the eye does not have to change its focus every time the position of the spot changes.

In yet another embodiment of the device according to the invention the spot can be positioned in a circular pattern by the controller. This pattern is especially preferred when making photo's of the fundus of the eye.

In still another embodiment of the device according to the invention the controller comprises a display for displaying the position of the spot on the screen. This display provides a feed back for the doctor when operating the device according to the invention. It enables the doctor to check whether the spot is positioned at the desired location and to check whether the patient is looking to the spot.

The invention also relates to a headrest comprising:
- a stand;
- a vertical frame arranged on the stand;
- a chin support arranged in the frame for supporting a patient's head; and
- a device for eye fixation according to the invention, mounted to the vertical frame and positionable in front of one of the eye's of a patient.

The headrest provides a stable support for the head of the patient. The patient lays the chin on the chin support such that the head of the patient does not move. It also makes it possible to position the device according to the invention at a stable position in front of one of the eyes.

In a preferred embodiment of the headrest according to the invention, the device for eye fixation is mounted on an arm, which is hingedly arranged to the frame. As a result the arm can be swung back to give access to the other eye. This is of advantage when both eyes are to be examined.

Another embodiment of the headrest according to the invention comprises a second device for eye fixation being mounted on a second arm, which is hingedly arranged to the frame.

It is also possible to arrange the device according to the invention movable on the frame, such that it can be moved in front of each eye as desired.

These and other features of the invention will be elucidated in conjunction with the accompanying drawings.
Figure 1 shows a perspective view of a fundus camera with a device according to the invention.
Figure 2 shows a perspective view of the device according to figure 1.
Figure 3 shows a cross sectional view of the device according to figure 1 and 2.
Figure 4 shows a top view of the controller of the device according to the previous figures.

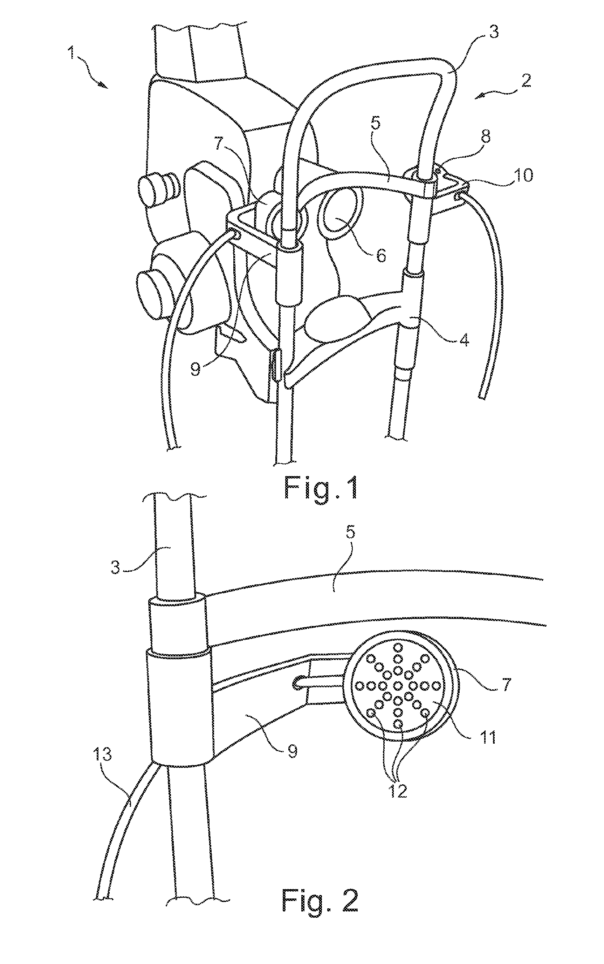

In figure 1 a fundus camera 1 is shown. A head rest 2 is arranged in front of the fundus camera 1. The head rest 2 has a frame 3 with a chin support 4. The frame 3 also comprises a forehead support 5. The head rest 2 makes sure that the head of a patient is stabilized relative to the fundus camera 1, such that steady photo's can be made.

The fundus camera 1 is positioned with the lens 6 in front of the eye to be examined of the patient. In front of the other eye, which is not examined, a device 7 for eye fixation is positioned. This device 7 is mounted to a bracket 9, which is arranged hingedly to the frame 3. A second device 8 for eye fixation is also arranged by a bracket 10 to the frame 3. This second device 8 is swung back to provide room for the lens 6 of the camera 1.

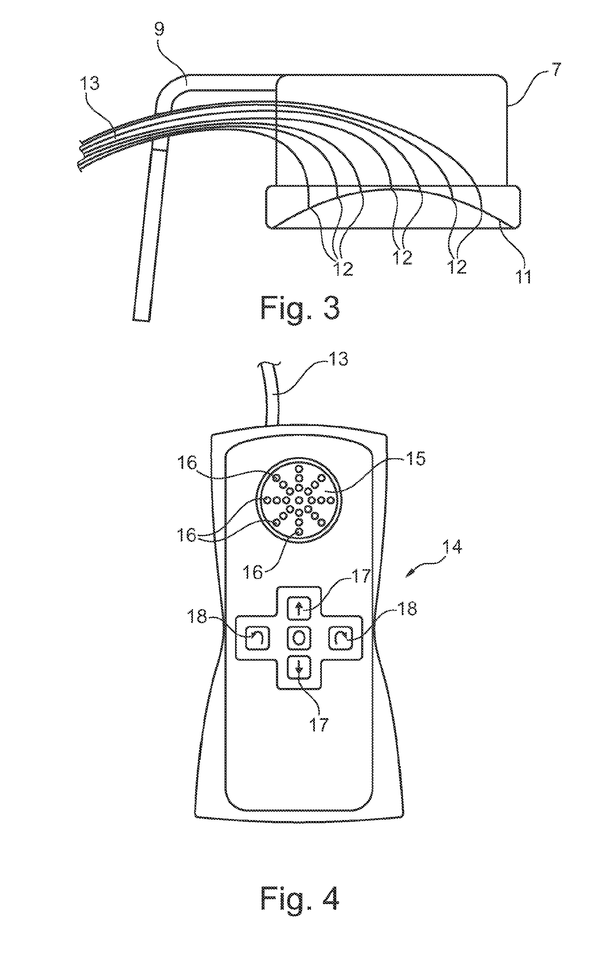

Figure 2 shows an enlarged view of the device 7 for eye fixation. This device 7 has a screen 11. In this screen 11 a number of optic fiber end 12 are arranged. The optic fibers 13 run through the bracket 9 and go to the controller 14 (see figure 4). When one of these optic fibers 13 is fed with light, a spot will be shown on the screen 11 on which the eye of the patient can focus.

Figure 3 shows a cross sectional view of the device 7. The screen 11 is concave to better accommodate an eye of a patient and also to ensure an equal distance between one of the optic fiber ends 12 and the eye of the patient.

Figure 4 shows the controller 14 in which the optic fibers 13 end. In this controller 14 light sources (not shown) are arranged, which send light through these fibers such that a spot is displayed on the screen 11 of the device 7.

The controller 14 comprises a display 15 with LED's 16 which correspond with the optic fiber ends 12. This enables a doctor to check whether light is sent through the correct optic fiber 13 and provides a spot on the screen 11 at the correct position, such that the patient will move the eyes to the desired position.

The controller 14 is furthermore provided with keys 17, 18 to move the spot to the desired position on the screen 11. The keys 17 are used to move the spot on the screen 11 up and down, while the keys 18 are used to rotate the spot clockwise and counter-clockwise.

With the device according to the invention it is possible to have a spot, like a light spot, in front of an eye of a patient, when the other eye is examined. The spot provides a stimulus for the non examined eye, such that the eye is attracted to this spot and both eyes are moved into the desired position.

The controller enables the doctor to move the spot around, without even having to look up from the examination device. Thus the position of the eyes of the patient can be changed, by moving the spot, without the need for the doctor to look away from the examination device or to stand up an manually rearrange a light, as is common in the prior art.

## Claims

1. Device for eye fixation, the device comprising:
- a screen arrangable in front of an eye of a patient;
- a controller for controlling the screen, wherein the screen is controlled to show a spot at a desired position for a patient's eye to fixate on.

2. Device according to claim 1, wherein the screen comprises a number of light sources connected to the controller for switching on and off the light sources.

3. Device according to claim 1 or 2, wherein the device comprises a number of optical fibers, which each have one end arranged in the screen and the other end connected to a light source arranged in the controller.

4. Device according to claim 1 or 2, where the device comprises a number of LED's arranged in the screen and connected to the controller.

5. Device according to any of the preceding claims, wherein the screen is concave.

6. Device according to any of the preceding claims, wherein the spot can be positioned in a circular pattern by the controller.

7. Device according to any of the preceding claims, wherein the controller comprises a display for displaying the position of the spot on the screen.

8. Headrest comprising:
- a stand;
- a vertical frame arranged on the stand;
- a chin support arranged in the frame for supporting a patient's head; and
- a device for eye fixation according to any of the preceding claims, mounted to the vertical frame and positionable in front of one of the eye's of a patient.

9. Headrest according to claim 8, wherein the device for eye fixation is mounted on an arm, which is hingedly arranged to the frame.

10. Headrest according to claim 8, comprising a second device for eye fixation being mounted on a second arm, which is hingedly arranged to the frame.
